# EUROPÄISCHE PATENTSCHRIFT

(11) **EP 0 982 016 B1**
(45) Veröffentlichungstag und Bekanntmachung des Hinweises auf die Patenterteilung: **15.10.2003**
(21) Anmeldenummer: 99115798.3
(22) Anmeldetag: 11.08.1999
(51) Int. Cl.: A61F 13/15

(54) **Verfahren und Vorrichtung zum Herstellen von mehrlagigen Hygieneprodukten**
Method and apparatus for producing multilayered sanitary articles
Procédé et dispositif pour la fabrication de produits hygiéniques multicouches

(30) Priorität: 25.08.1998 DE 19838494
(43) Veröffentlichungstag der Anmeldung: 01.03.2000
(73) Patentinhaber: Winkler + Dünnebier Aktiengesellschaft, 56562 Neuwied (DE)
(72) Erfinder: Geissen, Armin, 56581 Melsbach (DE); Becker, Albert, 56587 Strassenhaus (DE); Büntgen, Hans Peter, 56626 Andernach (DE); Wilms, Anja, 56581 Melsbach (DE); Zilles, Konrad, 56626 Andernach (DE); Söhn, Hans Werner, 56299 Ochtendung (DE)
(74) Vertreter: Schieferdecker, Lutz, Dipl.-Ing.

(56) Entgegenhaltungen:
- US-A- 3 607 578
- US-A- 3 984 272

## Beschreibung

Die Erfindung betrifft ein Verfahren und eine Vorrichtung zum Herstellen von mehrlagigen Hygieneprodukten mit den Merkmalen der Ansprüche 1 bzw. 8, wobei unterschiedliches und zum Teil bahnförmiges Ausgangsmaterial für die einzelnen Lagen jeweils zu Verarbeitungsstationen und in der Regel zu verschiedenartigen Verarbeitungsstationen einer Vorrichtung geführt sowie dort verarbeitet und zu einem Produkt verbunden wird.

Vorrichtungen der genannten Art sind bekannt und dienen beispielsweise zum Herstellen von Inkontinenzprodukten, Damenbinden, Babywindeln und dergleichen. Moderne Produkte dieser Art bestehen aus mehreren Teilen und sind sandwichartig aufgebaut. Dazu ist es notwendig, meist schmales, bahnförmiges Material auf großen Rollen zu verarbeiten, das den eigentlichen Verarbeitungsstationen in geeigneter Weise zugeführt wird. Die Rollenanordnungen für das bahnförmige Material befinden sich in der Regel über den Verarbeitungsstationen, damit sie gut zugänglich sind und das bahnförmige Material von neuen Vorratsrollen möglichst problemlos in den Verarbeitungsprozeß eingefügt werden kann. Trotz dieser kompakten Bauweise sind die bekannten Vorrichtungen der hier interessierenden Art oft mehr als 15 und bis 20 Meter lang. Einen derartigen Stand der Technik zeigt die US-A-3,607,578.

Grundsätzlich sind die hier interessierenden Vorrichtungen sehr geräuschintensiv und erzeugen einen derartigen Lärm, daß sie zur Reduzierung des Geräuschpegels gekapselt werden müssen. Die Gehäuse weisen allerdings großflächige Klappen auf, damit abgearbeitete Vorratsrollen durch neue ersetzt werden können. Da ferner die Arbeitsgeschwindigkeit von modernen Maschinen sehr hoch ist, ist es bereits nach kurzer Zeit wieder notwendig, die Gehäuseklappen zu öffnen und die abgearbeiteten Vorratsrollen durch neue zu ersetzen.

Der Austausch von Vorratsrollen macht es darüber hinaus notwendig, die Arbeitsgeschwindigkeit der Vorrichtung kurzfristig zu senken. Nur dann ist ein problemloses Anschließen des bahnförmigen Materials der jeweils nächsten Vorratsrolle an das Ende der letzten bzw. abgearbeiteten Vorratsrolle gewährleistet. Dennoch muß jeweils eine gewisse Menge der zum Übergangsbereich gehörenden Produkte ausgeschleust und entsorgt werden.

Der Erfindung liegt daher die Aufgabe zugrunde, Maßnahmen vorzusehen, damit die Vorrichtung über möglichst lange Zeit mit höchster Maschinenleistung arbeiten kann, wozu es notwendig ist, Vorratsrollen mit möglichst großem Durchmesser zu verwenden. Verbunden damit ist ein grundsätzlich pro Schicht zu minimierender Abfall bzw. Ausschuß, wobei auch der personelle Einsatz, der zur Bedienung der Vorrichtung erforderlich ist, möglichst niedrig gehalten werden soll.

Zur Lösung dieser Aufgabe sieht die Erfindung mit den Merkmalen des kennzeichnenden Teiles des Anspruches 1 vor, daß zumindest ein Teil der Ausgangsmaterialien von ihren Vorratsstationen aus in Richtung quer zur Verarbeitungsebene dieser zugeführt wird. Grundsätzlich wenigstens eine, zweckmäßigerweise aber möglichst viele oder alle Vorratsrollen sollen erfindungsgemäß in einer anderen Reihe beziehungsweise in einer anderen, das heißt parallelen Vertikalebene angeordnet sein als die zum Herstellen der Produkte dienenden Verarbeitungsstationen und diesen quer zur Verarbeitungsebene zugeführt werden. Dadurch ist es möglich, die Verarbeitungsstationen dauerhaft geräuschmindernd zu kapseln. Die in einer anderen Ebene angeordneten Vorratsstationen mit ihren Vorratsrollen brauchen wegen ihrer geringen Geräuschentwicklung überhaupt nicht mehr gekapselt zu werden, so daß dort einfache Sicherheitsmaßnahmen zum Schutz des Bedienungspersonals genügen.

Ferner können die Vorratsrollen bei Anordnung in einer eigenen Vorratsebene größer dimensioniert werden als bisher, wodurch sich die Standzeit der mit höchster Leistung arbeitenden Vorrichtung erhöht. Eine Verringerung des Ausschusses und eine Erhöhung der Produktion je Schicht ist insbesondere dann die Folge, wenn eine komplette Trennung zwischen der den Verarbeitungsstationen vorbehaltenen Verarbeitungsebene und der Vorratsebene erfolgt, in der das Ausgangsmaterial vorrätig gehalten wird.

Zweckmäßig ist es schließlich, wenn das Ausgangsmaterial von der Vorratsebene bodenseitig zu der Verarbeitungsebene geführt wird. Dies kann in einem Kanal oder unter einem Podest erfolgen. Ferner muß dies nicht nur für bahnförmiges Ausgangsmaterial gelten. Auch nicht bahnförmiges Ausgangsmaterial kann bodenseitig von einer Vorratsebene zu einer Verarbeitungsebene transportiert werden.

Die Erfindung ist aber nicht auf eine bodenseitige Überführung des Ausgangsmaterial beschränkt. Die Überführung kann grundsätzlich auch deckenseitig oder über Kopf erfolgen.

Nicht bahnförmiges oder sonstiges Ausgangsmaterial, das nicht auf Vorratsrollen angeordnet ist, kann ebenso in einer besonderen Vorratsebene vorrätig gehalten werden und von dort den Verarbeitungsstationen zugeführt werden. Derartiges, sonstiges Ausgangsmaterial können Flockenmaterial und/oder Super -Absorbent, das Quellstoffe zum Beispiel auf Acryl- oder Zellulose-Basis enthält, und/oder desodorierende Stoffe und/oder geruchshemmende Stoffe und/oder verbindende Stoffe wie zum Beispiel Leime oder dergleichen sein.

Die Anordnung von Vorratsrollen für bahnförmiges Ausgangsmaterial kann schließlich derart vorgesehen sein, daß die Vorratsrollen parallel zur Verarbeitungsebene und/oder quer zur Verarbeitungsebene angeordnet sind bzw. abgerollt werden.

Die Vorrichtung zur Durchführung des Verfahrens sieht erfindungsgemäß vor, daß die Verarbeitungsebene mit den Verarbeitungsstationen und die Vorratsebene mit den Vorratsstationen unterschiedliche Vertikalebenen sind. Für einen konkreten Anwendungsfall ist es zweckmäßig, wenn die Verarbeitungsebene und die Vorratsebene parallel zueinander angeordnete Vertikalebenen sind.

Ferner ist erfindungsgemäß vorgesehen, daß die Verarbeitungsebene mit den Verarbeitungsstationen und die Vorratsebene mit den Vorratsstationen nicht nur parallel zueinander, sondern auch mit deutlichem Abstand voneinander beziehungsweise nebeneinander angeordnet sind. Dies bedeutet in der Praxis, daß zweckmäßigerweise sogar ein Gang zwischen den parallel zueinander angeordneten Verarbeitungsstationen und Vorratsstationen vorgesehen sein kann.

Grundsätzlich kann allerdings auch die Vorratsebene mit den Vorratsstationen quer zu der die Verarbeitungsstationen aufweisenden Verarbeitungsebene stehen.

Schließlich sieht die Erfindung vor, daß nicht nur Vorratsstationen, in denen sich Vorratsrollen befinden, sondern auch sonstiges Ausgangsmaterial enthaltende Vorratsstationen in einer vertikalen Vorratsebene außerhalb der vertikal stehenden Verarbeitungsebene angeordnet sind, in der sich die zur Produktion dienenden Verarbeitungsstationen befinden.

Weitere Merkmale der Erfindung gehen aus Unteransprüchen hervor.

Die Erfindung wird nachstehend anhand von Ausführungsbeispielen, die in der Zeichnung dargestellt sind, näher beschrieben. Dabei zeigen die Figuren jeweils schematisch sowie mit zum Teil unterschiedlichem Maßstab:

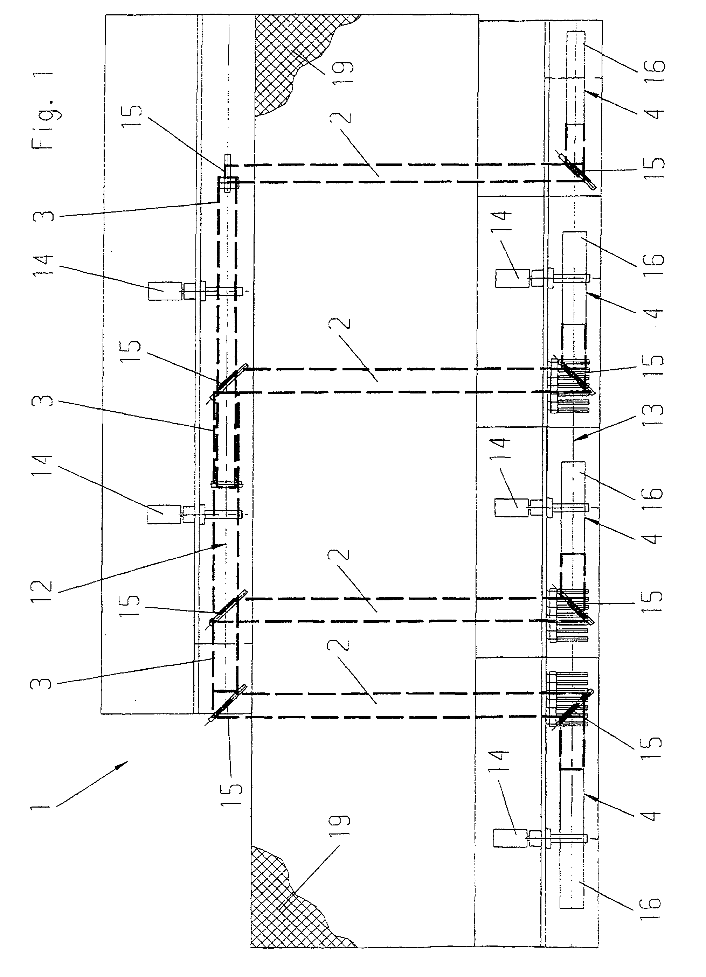

- Fig. 1:: eine Draufsicht auf Teile einer Vorrichtung;
- Fig. 2:: eine Seitenansicht mit verschiedenen Verarbeitungsstationen in der Verarbeitungsebene;
- Fig. 3:: eine Seitenansicht mit Vorratsstationen in einer Vorratsebene;
- Fig. 4:: eine Seitenansicht von anderen Vorratsstationen in der Vorratsebene und
- Fig. 5:: einen Schnitt durch eine Verarbeitungsstation in ihrer Verarbeitungsebene und durch eine Vorratsstation in der Vorratsebene.

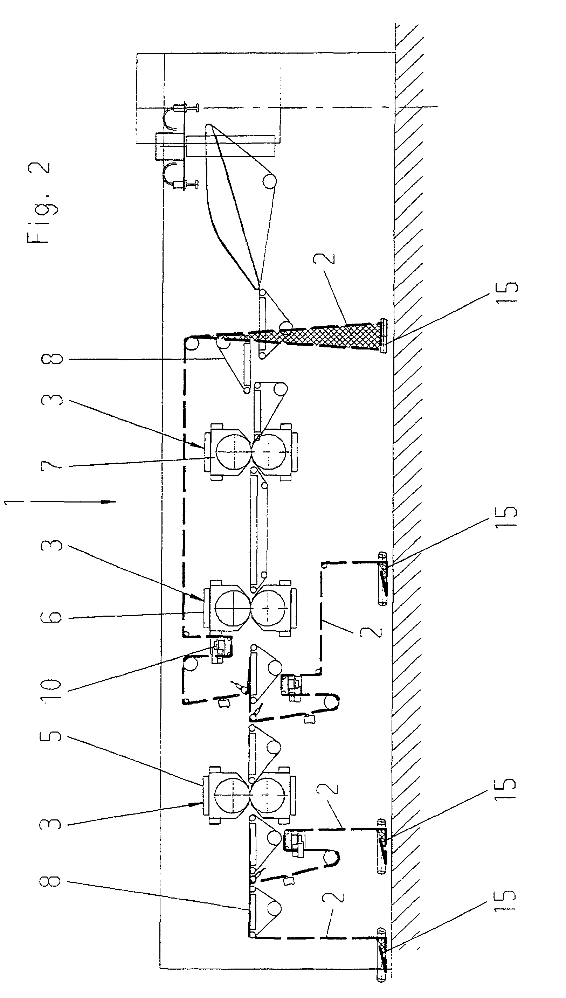

Eine Vorrichtung 1 zum Herstellen von mehrlagigen Hygieneprodukten, wobei bahnförmiges Ausgangsmaterial 2 oder auch sonstiges Ausgangsmaterial für die einzelnen Lagen benötigt wird, umfaßt in der Regel mehrere Verarbeitungsstationen 3 sowie mehrere Vorratsstationen 4. Bei den Verarbeitungsstationen kann es sich um Prägestationen 5 und 6 sowie um eine Schneidstation 7 gemäß Fig. 2 handeln, denen ferner Transporteinrichtungen 8, Einrichtungen 9 zum Auftragen von Leim oder Klebemittel sowie eine Einrichtung 10 zur Bahnsteuerung zugeordnet sind. Einzelheiten dieser Verarbeitungsstationen 3 sind im vorliegenden Fall ohne Interesse. Entscheidend ist allein die vollständige oder überwiegend vorgesehene, örtliche Trennung der zum Herstellen der Produkte dienenden Verarbeitungsstationen 3 von mindestens einer zum Zuführen von bahnförmigem Ausgangsmaterial 2 dienenden Vorratsstation 4 durch Anordnung in verschiedenen Reihen oder Ebenen 12, 13. Zweckmäßigerweise bzw. gemäß den in den Fig. dargestellten Ausführungsbeispielen sind die Ebenen 12 und 13 parallel zueinander angeordnet. Die Ebene 12 mit den Verarbeitungsstationen 3 wird nachstehend auch als Verarbeitungsebene 12 und die Ebene 13 mit den Vorratsstationen 4 als Vorratsebene 13 bezeichnet.

Antriebe 14 und Umlenkeinrichtungen 15 für das bahnförmige Ausgangsmaterial 2 gehören ebenso zu den Verarbeitungsstationen 3 wie zu den Vorratsstationen 4.

Das bahnförmige Ausgangsmaterial 2 befindet sich in den Vorratsstationen 4 jeweils auf Vorratsrollen 16 und durchläuft dort einen grundsätzlich bekannten Materialbahn-Speicher 17. Vor dem Materialbahn-Speicher befindet sich eine Einrichtung 18 zum Verbinden von Bahnenden. In Laufrichtung hinter dem Materialbahn-Speicher 17 wird das bahnförmige Ausgangsmaterial 2 gemäß den in den Figuren dargestellten Ausführungsbeispielen zu einer bodenseitig angeordneten Umlenkeinrichtung 15 geführt.

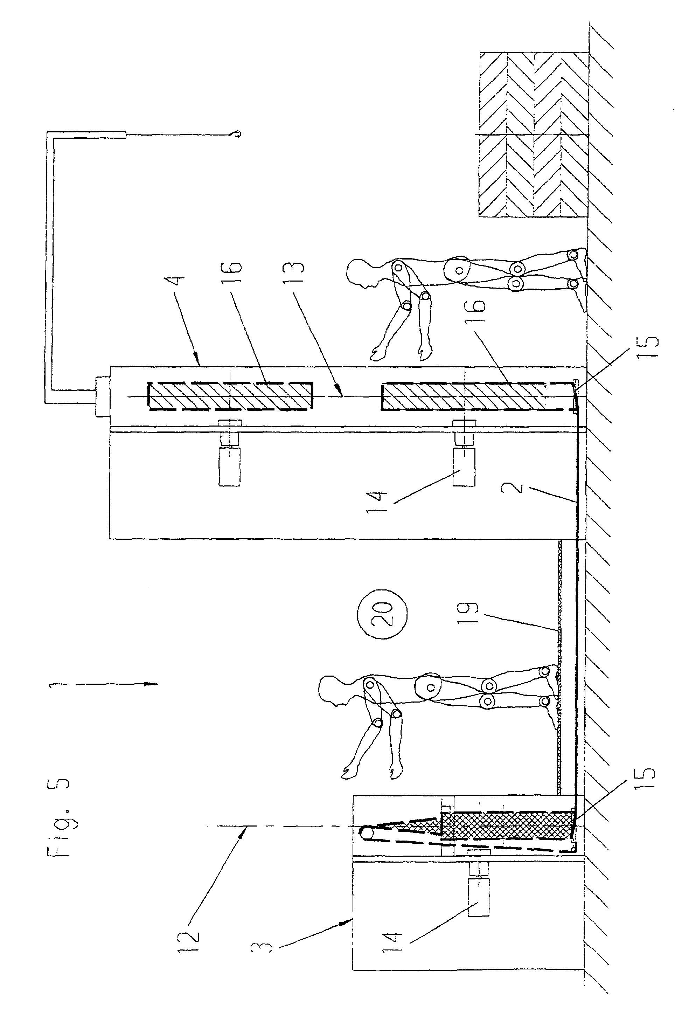

Von den Umlenkeinrichtungen 15, die zu den Vorratsstationen 4 gehören, gelangt das bahnförmige Ausgangsmaterial 2 bodenseitig in einem Kanal oder unter einem Podest 19 bzw. unter eine Lauffläche für Bedienungspersonal zu den Umlenkeinrichtungen 15 auf der Seite der Verarbeitungsstationen 3 (Fig. 5). Das Podest 19 befindet sich dabei in einem Gang 20 zwischen den Verarbeitungsstationen 3 und den Vorratsstationen 4.

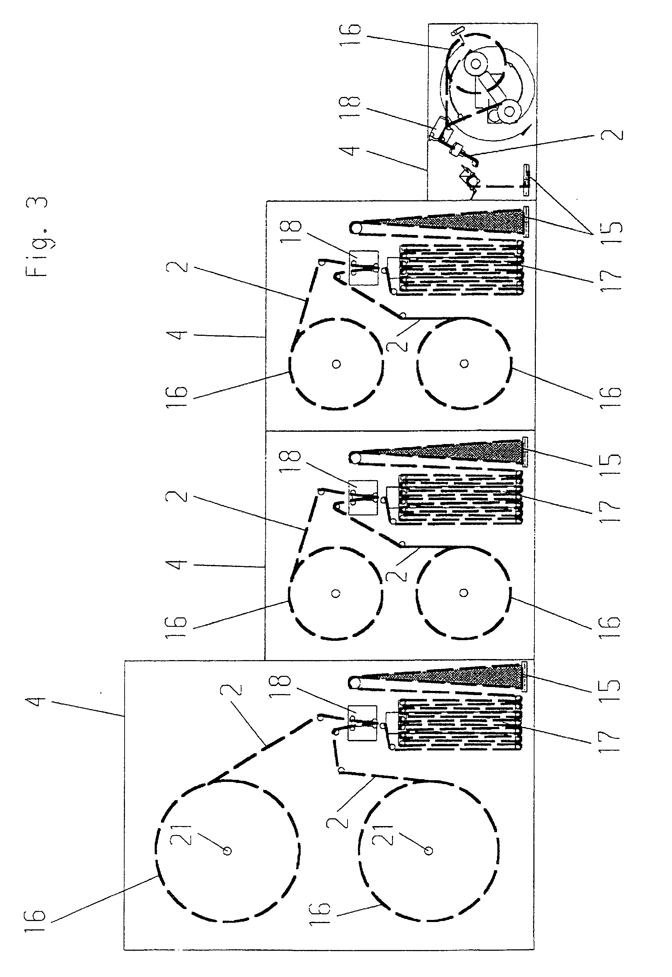

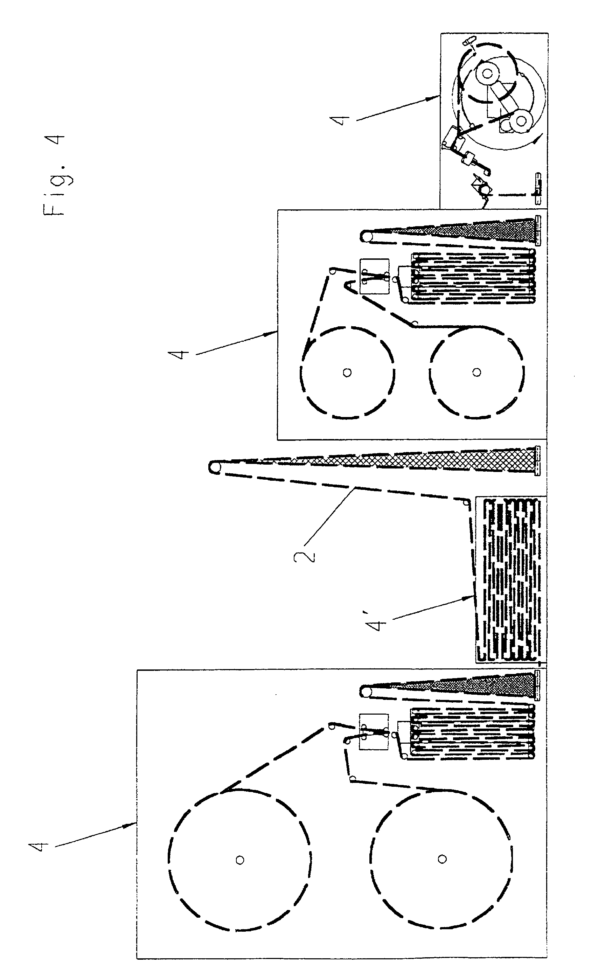

Eine abgewandelte Ausführungsform mit Vorratsstationen 4 wie in Fig. 3 und einer Vorratsstation 4' sind in Fig. 4 dargestellt. Die Vorratsstation 4' weist statt Vorratsrollen eine Materialbox für das bahnförmige Ausgangsmaterial 2 auf. Derartige Materialboxen 4' sind grundsätzlich bekannt.

Die Erfindung ist nicht auf bestimmte Verarbeitungsstationen 3 oder Vorratsstationen L bzw. 4' gerichtet. Wesentlich ist, daß die Ebenen 12 und 13, in denen sich einzelne oder Gruppen von Verarbeitungsstationen 3 bzw. von Vorratsstationen 4, 4' befinden, nebeneinander bzw. parallel zueinander angeordnet sind. Dies gilt für die Ebene 13 mit bahnförmigem Ausgangsmaterial 2. Aber auch nicht bahnförmiges Ausgangsmaterial kann in einer anderen Ebene angeordnet sein als die Verarbeitungsstationen 3.

Die Figuren zeigen konkret nur den Fall, daß die Vorratsrollen 16 mir Ihren Achsen 21 (Fig. 3) und folglich die Vorratsstationen 4 in einer Reihe hintereinander entsprechend der Vorratsebene 13 sowie parallel und im Abstand zu den Verarbeitungsstationen 3 angordnet sind. Die Vorratsstationen 4 können aber auch zum Teil oder insgesamt rechtwinklig zu der Ebene der Verarbeitungsstationen 3 angordnet sein. Auch dann befinden sich alle oder einige Vorratsstationen 4 in einer Ebene, die verschieden ist von der Ebene 12 der Verarbeitungsstationen 3.

## Patentansprüche

1. Verfahren zum Herstellen von mehrlagigen Hygieneprodukten, bei dem bahnförmige Ausgangsmaterialien (2) für die einzelnen Lagen aus einer Reihe von Vorratsstationen (4), die in einer vertikalen Vorratsebene (13) angeordnet sind, einer Reihe von hintereinander in einer vertikalen Verarbeitungsebene (12) angeordneten Verarbeitungsstationen (3) zugeführt und dort verarbeitet sowie zu dem Produkt verbunden werden, **dadurch gekennzeichnet, dass** zumindest ein Teil der Ausgangsmaterialien (2) von ihren Vorratsstationen (4) aus in Richtung quer zur Verarbeitungsebene (12) dieser zugeführt wird.

2. Verfahren nach Anspruch 1, **dadurch gekennzeichnet, dass** die Ausgangsmaterialien (2) von Vorratsrollen (16) mit quer zur vertikalen Vorratsebene (13) verlaufenden Achsen zugeführt werden.

3. Verfahren nach Anspruch 2, **dadurch gekennzeichnet, dass** auch andere aufgerollte bahnförmige Ausgangsmaterialien, insbesondere Flockenmaterial und/oder Super-Absorbent, das Quellstoffe zum Beispiel auf Acryl- oder Zellulosebasis enthält, und/oder desodorierende Stoffe und/oder geruchshemmende Stoffe und/oder verbindende Stoffe wie Leime, in Richtung quer zur Verarbeitungsebene (12) dieser zugeführt werden.

4. Verfahren nach einem der Ansprüche 1 bis 3, **dadurch gekennzeichnet, dass** die Ausgangsmaterialien (2) bodenseitig von der Vorratsebene (13) zur Verarbeitungsebene (12) geführt werden.

5. Verfahren nach einem der Ansprüche 1 bis 3, **dadurch gekennzeichnet, dass** die Ausgangsmaterialien (2) deckenseitig von der Vorratsebene (13) zur Verarbeitungsebene (12) geführt werden.

6. Vorrichtung zur Herstellung von mehrlagigen Hygieneprodukten gemäß dem Verfahren nach Anspruch 1 mit einer Reihe von hintereinander in einer vertikalen Verarbeitungsebene (12) angeordneten Verarbeitungsstationen (3) und mit einer Reihe von in einer vertikalen Vorratsebene (13) angeordneten Vorratsstationen (4) für bahnförmige Ausgangsmaterialien (2), **dadurch gekennzeichnet, dass** die Verarbeitungsebene (12) mit den Verarbeitungsstationen (3) und die Vorratsebene (13) mit den Vorratsstationen (4) unterschiedliche Vertikalebenen sind.

7. Vorrichtung nach Anspruch 6, **dadurch gekennzeichnet, dass** die Verarbeitungsebene (12) mit den Verarbeitungsstationen (3) und die Vorratsebene (13) mit den Vorratsstationen (4) parallel und mit Abstand nebeneinander angeordnet sind.

8. Vorrichtung nachAnspruch 6, **dadurch gekennzeichnet, dass** die Vorratsebene (13) mit den Vorratsstationen (4) quer zur Verarbeitungsebene (12) mit den Verarbeitungsstationen (3) verläuft, wobei die Vorratsstationen (4) im Seitenabstand zur Verarbeitungsebene (12) angeordnet sind.

## Claims

1. Process for the production of multilayered hygiene products, in which web-shaped starting materials (2) for the individual layers are fed from a row of stock stations (4), which are arranged in a vertical stock plane (13), to a row of processing stations (3) arranged one after the other in a vertical processing plane (12) and processed there and are bound to form the product, **characterized in that** at least one part of the starting materials (2) is fed out from their stock stations (4) in a direction transverse to the processing plane (12) thereof.

2. Process according to claim 1, **characterized in that** the starting materials (2) are fed from stock rollers (16) with axes running transverse to the vertical stock plane (13).

3. Process according to claim 2, **characterized in that** other rolled-up web-shaped starting materials, in particular flock materials and/or super absorbent, which contain swelling agents based for example on acrylic or cellulose, and/or deodorizing substances and/or odour-inhibiting substances and/or bonding substances such as glues, are also fed in a direction transverse to the processing plane (12) thereof.

4. Process according to one of claims 1 to 3, **characterized in that** the starting materials (2) are fed bottom-side from the stock plane (13) to the processing plane (12).

5. Process according to one of claims 1 to 3, **characterized in that** the starting materials (2) are fed top-side from the stock plane (13) to the processing plane (12).

6. Device for the production of multilayered hygiene products according to the process according to claim 1 with a row of processing stations (3) arranged one after the other in a vertical processing plane (12) and with a row of stock stations (4) arranged in a vertical stock plane (13) for web-shaped starting materials (2), **characterized in that** the processing plane (12) with the processing stations (3) and the stock plane (13) with the stock stations (4) are different vertical planes.

7. Device according to claim 6, **characterized in that** the processing plane (12) with the processing stations (3) and the stock plane (13) with the stock stations (4) are arranged parallel and spaced apart alongside each other.

8. Device according to claim 6, **characterized in that** the stock plane (13) with the stock stations (4) runs transverse to the processing plane (12) with the processing stations (3), the stock stations (4) being arranged at a lateral distance from the processing plane (12).

## Revendications

1. Procédé pour la fabrication de produits hygiéniques multi-couches, où des matériaux de départ (2) en forme de bande pour les couches individuelles sont amenés d'une rangée de postes de réserve (4) qui sont disposés dans un plan de réserve verticale (13), à une rangée de postes de traitement (3) disposés les uns derrière les autres dans un plan de traitement vertical (12) et y sont traités et reliés pour former le produit, **caractérisé en ce qu'**au moins une partie des matériaux de départ (2) est amenée de ses postes de réserve (4) en direction transversalement au plan de traitement (12) à ceLui-ci.

2. Procédé selon la revendication 1, **caractérisé en ce que** les matériaux de départ (2) sont amenés à partir de rouleaux de réserve (16) avec des axes s'étendant transversalement au plan de réserve vertical (13).

3. Procédé selon la revendication 2, **caractérisé en ce qu'**aussi d'autres matériaux de départ en forme de bandes enroulées, notamment du matériau floconneux et/ou super-absorbant, qui contient des agents de gonflement, par exemple à base d'acryle ou de cellulose et/ou des agents déodorisants et/ou des agents retardant les odeurs et/ou des agents de liaison comme des colles, en direction transversalement au plan de traitement (12) à celui-ci.

4. Procédé selon l'une des revendications 1 à 3, **caractérisé en ce que** les matériaux de départ (2) sont amenés, côté fond, du plan de réserve (13) au plan de traitement (12) .

5. Procédé selon l'une des revendications 1 à 3, **caractérisé en ce que** les matériaux de départ (2) sont amenés, côté plafond, du plan de réserve (13) au plan de traitement (12).

6. Dispositif pour la fabrication de produits hygiéniques multi-couches conformément au procédé selon la revendication 1, avec une série de postes de traitement (3) disposés les uns derrière les autres dans un plan de traitement vertical (12) et une série de postes de réserve (4) disposés dans un plan de réserve vertical (13) pour des matériaux de départ (2) en forme de bande, **caractérisé en ce que** le plan de traitement (12) avec les postes de traitement (3) et le plan de réserve (13) avec les postes de réserve (4) sont des plans verticaux différents.

7. Dispositif selon la revendication 6, **caractérisé en ce que** le plan de traitement (12) avec les postes de traitement (3) et le plan de réserve (13) avec les postes de réserve (4) sont disposés parallèlement et à une distance l'un à côté de l'autre.

8. Dispositif selon la revendication 6, **caractérisé en ce que** le plan de réserve (13) avec les postes de réserve (4) s'étend transversalement au plan de traitement (12) avec les postes de traitement (3), où les postes de réserve (4) sont disposés à une distance latérale du plan de traitement (12).
